## Europäisches Patentamt
## European Patent Office
## Office européen des brevets

(19)

(11) Veröffentlichungsnummer: **0 313 805 B1**

(12) **EUROPÄISCHE PATENTSCHRIFT**

(45) Veröffentlichungstag der Patentschrift:
10.07.91 Patentblatt 91/28

(51) Int. Cl.⁵: **G01N 31/22, G01N 33/18**

(21) Anmeldenummer: **88115373.8**

(22) Anmeldetag: **20.09.88**

(54) **Indikatoren und Verfahren zum Anzeigen von organischen Verbindungen in Wasserströmen.**

(30) Priorität: **30.09.87 DE 3733034**

(43) Veröffentlichungstag der Anmeldung:
**03.05.89 Patentblatt 89/18**

(45) Bekanntmachung des Hinweises auf die
Patenterteilung:
**10.07.91 Patentblatt 91/28**

(84) Benannte Vertragsstaaten:
**CH DE FR GB IT LI**

(56) Entgegenhaltungen:
**DE-A- 3 412 023**
**GB-A- 1 311 324**
**THE JOURNAL OF THE AMERICAN CHEMI-
CAL SOCIETY, Band 69, 1947 BAUMANN; EI-
CHORN "Fundamental Properties of a
Synthetic Cation Exchange Resin" Seiten
2820-2836**

(73) Patentinhaber: **BAYER AG**
**W-5090 Leverkusen 1 Bayerwerk (DE)**

(72) Erfinder: **Mitschker, Alfred, Dr.**
**Am Gartenfeld 50**
**W-5068 Odenthal (DE)**
Erfinder: **Hartung, Sigurd, Dr.**
**verstorben (DE)**
Erfinder: **Klein, Harald**
**Opladener Strasse 3**
**W-4018 Langenfeld (DE)**
Erfinder: **Gräfe, Andreas**
**Odenthaler Markweg 60**
**W-5090 Leverkusen 1 (DE)**

## Beschreibung

Die Erfindung betrifft die Verwendung bestimmter Polymerisate als Indikatoren für in Wasserströme geratene organische Verbindungen und ein Verfahren zum Anzeigen in Wasserströmen befindlicher organischer Verbindungen.

Für die Reinerhaltung der Gewässer ist von großer Bedeutung, daß Leckagen in industriellen und gewerblichen Anlagen, durch die organische Verbindungen in in Rohrleitungen geführte Wasserströme und weiter in Gewässer gelangen können, schnellstens erkannt werden, um die meist unerkannt eingetretenen Leckagen so schnell wie möglich beseitigen zu können.

Um zu gewährleisten, daß die aus gewerblichen Anlagen abfließenden Wasserströme keine organischen Verbindungen als Verunreinigungen enthalten, werden diese Wasserströme fortlaufend auf etwa in ihnen enthaltene organische Verbindungen überwacht. Zur Erkennung von in Wasserströmen gegebenenfalls als Verunreinigungen auftretenden organischen Verbindungen werden heute folgenden Verfahren angewendet :

Messung der Wassertrübung mit einem Trübungsmesser (Nephelometer) ; das Verfahren hat den Nachteil, daß es nicht universell, d.h. zur Erkennung aller organischen Verbindungen anwendbar und im übrigen zu unempfindlich ist. Eine Trübung des Wassers durch die organische Verbindung bzw. die organischen Verbindungen tritt nur ein, wenn die Löslichkeit der organischen Verbindung(en) im betreffenden Wasserstrom überschritten ist und sich ein mehrphasiges System ausgebildet hat.

Bestimmung des Gesamtgehaltes an organischem, d.h. verbrennbarem Kohlenstoff einer Wasserprobe mittels Verbrennungsanalyse ; das Verfahren hat den Nachteil, daß es zu langwierig und aufwendig ist.

Ausblasen der in einer Wasserprobe enthaltenen organischen Verbindungen mit Luft und Bestimmung der durch das Ausblasen in die Gasphase überführten organischen Verbindungen mit Hilfe eines Flammenionisationsdetektors. Dieses Verfahren hat den Nachteil, daß es aufwendig und nicht universell anwendbar ist, da in ihm nur solche organischen Verbindungen erkennbar sind, die aus Wasser strippbar sind, diese Eigenschaft aber nur ein Teil der als Verunreinigungen in Wasserströmen vorkommenden organischen Verbindungen aufweist. .

Eine weitere Möglichkeit der Erkennung von Wasserverunreinigungen besteht in der sinnlichen Wahrnehmung von Geruch oder Farbe bzw. durch die Beobachtung der Auswirkungen, die der betreffende Wasserstrom bzw. die in ihm als Verunreinigungen enthaltenden organischen Verbindungen auf empfindliche Kleinlebewesen, z.B. auf Daphnien hat. Die kontinuierliche Überwachung von Wasserströmen mit Hilfe solcher biologischen Systeme ist jedoch z.Zt. noch nicht realisierbar.

Es wurde nun gefunden, daß sich mit Hilfe bestimmter Polymerisate in Wasserströmen befindliche organische Verbindungen zuverlässig und auf eine sehr einfache, schnelle Weise erkennen lassen. Es wurde gefunden, daß, wenn man den gegebenenfalls organische Verbindungen enthaltenden Wasserstrom, bzw. einen Teilstrom desselben, mit bestimmten quellfähigen organischen Polymerisaten in Berührung bringt, z.B. durch ein mit diesen Polymerisaten gefülltes Rohr leitet, sich die durch die Quellung des Harzes verursachte Änderung des Harzvolumens, bzw. des Druckes im Harz bzw. die durch diese Quellung im Harz verursachte Änderung des Strömungswiderstandes des Harzes für eine sehr genaue, empfindliche, reproduzierbare Anzeige etwa im Wasserstrom enthaltener organischer Verbindungen eignet.

Überraschenderweise genügen schon geringe Mengen von 20 bis 2000 ppm in Wasserstrom befindlicher organischer Verbindungen, um das Polymerisat in kurzer Zeit soweit anzuquellen, daß die durch die Quellung verursachte Volumenänderung, bzw. bei konstant gehaltenem Volumen, die verursachte Druckänderung oder die als Folge der Druckänderung eintretende Änderung des Strömungswiderstandes des Harzes, für eine eindeutige reproduzierbare Anzeige der organischen Verbindungen ausreicht.

Es ist bekannt, daß Polymerisate, z.B. solche auf Basis von mit Divinylbenzol vernetztem Polystyrol, in organischen Lösungsmitteln wie Toluol quellen (siehe z.B. Ullmann Encyclopädie der technischen Chemie, 2. Auflage, Band 13, Seite 297 ; J. Am. Chem. Soc. Vol 69, 2830 (1974)).

Da die Volumenänderungen, die durch kleine Mengen organischer Verbindungen, wie sie z.B. durch Leckagen in Wasserströme gelangen, verursacht werden, sehr klein und deshalb schwierig zu reproduzieren sind, war man bislang der Auffassung, daß das Quellvermögen von Polymerisaten in Gegenwart von organischen Verbindungen für ein Erkennen dieser Verbindungen in Wasserströmen nicht verwendbar ist.

Überraschenderweise wurde gefunden, daß die Quellung bestimmter Polymerisate in Gegenwart von kleinen Mengen organischer Verbindungen durchaus für eine zuverlässige, reproduzierbare Anzeige der organischen Verbindungen in Wasserströmen nutzbar ist.

Die Erfindung betrifft daher die Verwendung von bestimmten quellfähigen, organischen Polymerisaten als Indikatoren für in Wasserströmen befindliche organische Verbindungen.

Für die Anzeige der organischen Verbindungen in den Wasserströmen kann man unmittelbar die durch die Quellung bewirkte Volumenänderung der Indikatorharze verwenden. Um reproduzierbare Anzeigen zu erhalten, müssen die Volumenänderungen allerdings an sehr kleinen Harzmengen mit Hilfe äußerst empfindlicher Anzeigegeräte bestimmt werden.

Als vorteilhafter und wesentlich einfacher hat sich erwiesen, zur Anzeige organischer Verbindungen die durch die Quellung verursachte Änderung des Druckes bei konstant gehaltenem Volumen des Indikatorharzes oder – vorzugsweise – die Änderung des Strömungswiderstandes zu verwenden, den ein bestimmtes vom zu untersuchenden Wasserstrom durchflossenes Volumen des Indikatorharzes diesem Strom bietet.

Es wurde gefunden, daß die durch die Quellung verursachte Druckänderung innerhalb der Harzmasse, insbesondere aber die Änderung des Strömungswiderstandes der Harzmasse alle an eine reproduzierbare Anzeige gestellten Anforderungen erfüllen und sich außerdem auf einfache Weise zur Betätigung von Alarmsystemen eignen.

Die Erfindung betrifft ferner ein Verfahren zum Anzeigen von in Wasserströmen befindlichen organischen Verbindungen ; das Verfahren ist dadurch gekennzeichnet, daß man den zu untersuchenden Wasserstrom oder – vorzugsweise – einen Teilstrom desselben, durch ein mit einem quellfähigen organischen Polymerisat gefülltes Rohr leitet und die durch die Quellung des Polymerisates verursachte Änderung des Druckes in der Harzmasse oder die durch die Quellung verursachte Änderung des Strömungswiderstandes der Harzmasse für den Wasserstrom unmittelbar oder mittelbar bestimmt und/oder für eine Signalgebung ausnutzt.

Das erfindungsgemäße Verfahren eignet sich zum Erkennen von organischen Verbindungen, die organische Polymerisate anquellen. Organische Verbindungen, die diese Eigenschaften aufweisen sind z.B. :

Polare organische Verbindungen wie halogenierte aromatische Kohlenwasserstoffe, z.B. Chlorbenzol, Dichlorbenzol ; halogenierte aromatische Verbindungen z.B. Chlorbenzylchloride wie 2,4-Dichlorbenzylchlorid, Chlorbenzalchloride wie 2,4-Dichlorbenzalchlorid, Chlorbenzotrichloride ; chlorierte aliphatische Kohlenwasserstoffe z.B. Chloroform, Tetrachlormethan, Methylenchlorid, Dichlorethan ; Nitroaromaten z.B. Nitrobenzol, Nitrochlorbenzol ; Aniline, z.B. Anilin, Chloraniline, Nitroaniline ; aromatische Säurechloride z.B. Benzoylchlorid, 2,4-Dichlorbenzoylchlorid ; aromatische Aldehyde, z.B. Benzaldehyd, o-Chlorbenzaldehyd ; aliphatische Aldehyde mit einer C-Zahl > 4, z.B. i-Pentanal ; aliphatische Alkohole mit einer C-Zahl > 4, z.B. Butanol, Isoamylalkohol, Hexanol, Cyclohexanol ; aliphatische Ketone mit einer C-Zahl > 4, z.B. Methylisobutylketon ; Ester aliphatischer und aromatischer Carbonsäuren, z.B. Essigsäuremethylester, Essigsäurebutylester, Benzoesäureethylester ; Ether, z.B. Tetrahydrofuran.

Unpolare organische Verbindungen, z.B. aliphatische Kohlenwasserstoffe, wie n-Heptan und Benzine und aromatische Kohlenwasserstoffe wie Benzol, Toluol, Xylol, Ethylbenzol und Naphthalin.

Als quellfähige organische Polymerisate (Indikatorharze) eignen sich für das erfindungsgemäße Verfahren alle nicht ionogenen Polymerisate, die durch die im Wasserstrom zu erwartenden organischen Verbindungen angequollen werden. Das Quellvermögen der Polymerisate und damit ihre Eignung zum Nachweis der betreffenden organischen Verbindung kann durch folgenden einfachen Test ermittelt werden :

Ein 25 ml-Meßzylinder wird bis zur 10 ml-Markierung mit dem zu prüfenden Polymerisat gefüllt. Anschließend wird das Polymerisat mit der organischen Verbindung oder mit dem Gemisch organischer Verbindungen, die bzw. das vom Polymerisat angezeigt werden soll, übergossen, bis es vollständig von ihr bzw. von ihm bedeckt ist. Nach 24-stündigem Aufbewahren bei Raumtemperatur wird am Meßzylinder das Volumen abgelesen, auf das sich das Polymerisat in der organischen Verbindung ausgedehnt hat. Beträgt die Volumenzunahme $\Delta V > 10$ Volumen-%, ist das Polymerisat als Indikator für die betreffende organische Verbindung bzw. das Gemisch organischer Verbindungen geeignet.

Als Indikatorharze haben sich feinteilige Polymerisate auf der Basis von nicht oder nur schwach vernetztem Polyvinylchlorid, Polyacrylnitril, Poly(meth)acrylsäureestern und insbesondere von Polystyrol bewährt. Die feinteiligen Polymerisate können in Form von Pulvern, Granulaten und Perlpolymerisaten vorliegen ; bevorzugt sind Perlpolymerisate. Die Teilchengröße der feinteiligen Polymerisate sollte 0,15-1,25 mm, vorzugsweise 0,3 bis 0,8 mm betragen.

Als Indikator-Harze für die vorstehend genannten polaren organischen Verbindungen haben sich besonders mit 0 bis 4 Gew.-%, vorzugsweise mit 0 bis 2 Gew.-% Divinylbenzol, bezogen auf das Gesamtgewicht der Monomeren, vernetzte Polystyrol-Polymerisate bewährt. Für die vorstehend genannten unpolaren organischen Verbindungen eignen sich mit 0 bis 10 Gew.-%, vorzugsweise 0 bis 4 Gew.-% Divinylbenzol, bezogen auf das Gesamtgewicht der Monomeren, vernetzte Polyacrylsäureester langkettiger Alkohole.

Die bevorzugt für das erfindungsgemäße Anzeigen der organischen Verbindungen in Wasserströmen ausgenutzte Änderung des Strömungswiderstandes des vom zu untersuchenden Wasserstrom durchflossenen Indikatorharzes ist auf verschiedene Weise bestimmbar und für eine Signalgebung ausnutzbar. So läßt sich die Änderung des Strömungswiderstandes z.B. als Änderung des Druckes ($\Delta p$) bestimmen, die sich auf der Eintrittsseite des zu untersuchenden Wasserstromes in das mit Indikatorharz gefüllte Rohr einstellt. Die Änderung des Strömungswiderstandes kann ferner als Änderung (Abnahme) der Durchflußrate des Wasserstromes auf der Austrittsseite des zu untersuchenden Wasserstromes aus dem mit Indikatorharz gefüllten Rohr bestimmt werden. Eine weitere Möglichkeit zur Bestimmung des Zunahme des Strömungswiderstandes besteht darin, die Änderung der elektrischen Leistungsaufnahme einer Pumpe zu registrieren, welche den zu untersuchenden Wasserstrom mit konstanter Durchflußrate durch das mit Indikatorharz gefüllte Rohr drückt.

Die Änderung des Strömungswiderstandes ist, unabhängig davon ob sie als Druckänderung, als Änderung der Durchflußrate oder als Änderung der elektrischen Leistungsaufnahme der Pumpe registriert wird, abhängig vom Durchmesser des Rohres, in dem sich das Indikatorharz befindet und von der Stärke des durchfließenden Wasserstromes, d.h. der Durchflußmenge.

Es hat sich gezeigt, daß die Empfindlichkeit der Anzeige durch die Wahl des Verhältnisses Rohrdurchmesser (D) [mm]/Durchflußrate (F) [l/h] in dem Sinne bestimmt wird, daß die Empfindlichkeit umso größer ist, je kleiner der Wert des Quotienten D/F ist. Zum Beispiel wird eine bestimmte vorgegebene (z.B. für das Auslösen einer Alarmanlage erforderliche) Änderung des Strömungswiderstandes (Druckanstieg) $\Delta$p innerhalb einer vorgegebenen Ansprechzeit (z.B. 4 Minuten nach Auftreten der organischen Verbindung im Wasserstrom) erreicht bei einem D/F-Verhältnis von 0,4 bei einem Gehalt des Wasserstroms von 20 ppm organischer Verbindung, bei einem D/F-Verhältnis von 0,6 bei einem Gehalt des Wasserstroms von 200 ppm organischer Verbindung und

bei einem D/F-Verhältnis von 0,8 bei einem Gehalt des Wasserstroms von 2000 ppm organischer Verbindung.

Zum Erreichen der für das Erkennen erforderlichen Druck-, Durchflußraten- oder Leistungsaufnahmedifferenzen innerhalb möglichst kurzer Ansprechzeit hat es sich deshalb bewährt, den Durchmesser D des mit dem Indikatorharz gefüllten Rohres und die Stärke des durchfließenden Wasserstroms (Durchflußrate F) so aufeinander abzustimmen, daß der Quotient D/F < 1 ist und vorzugsweise 0,2 bis 0,8, besondere bevorzugt 0,4 bis 0,8 beträgt.

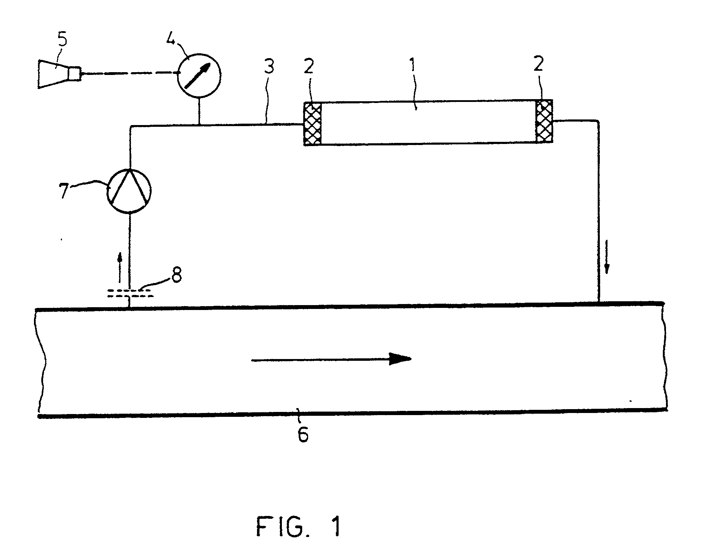

Das erfindungsgemäße Verfahren kann wie folgt ausgeführt werden (die Zahlen beziehen sich auf die in Fig. 1 angegebenen Bezugszeichen) :

Das die Anwesenheit organischer Verbindungen im Wasserstrom anzeigende Polymerisat ist in einem Rohr (1) angeordnet. Das Rohr (1) ist an seinen beiden Enden mit flüssigkeitsdurchlässigen, aber für das Polymerisat nicht durchlässigen Vorrichtungen (2), z.B. engmaschigen Sieben, Fritten u.s.w. versehen. Das Rohr (1) kann senkrecht oder waagerecht angeordnet sein. Vor dem Eintritt des Wasserstroms (3) in das Rohr (1) ist ein empfindliches Druckmeßgerät (4) angebracht. Gemessen wird vorteilhaft nicht der Absolutdruck, sondern die Druckdifferenz, die sich zwischen einem mit Indikatorharz gefüllten und von reinem Wasser durchflossenen Rohr und dem mit Indikatorharz gefüllten Rohr einstellt, das von dem von mit organischen Verbindungen verunreinigten Wasser durchflossen wird. Die Druckdifferenzen $\Delta$p liegen im Bereich von 0,01 bis 0,5 bar ; die Empfindlichkeit des Druckmeßgerätes (4) sollte deshalb in diesem Bereich liegen.

An die Stelle der unmittelbaren Registrierung der Druckdifferenzen kann z.B. auch das Druckmeßgerät mit einer Warnanlage (5) verbunden sein. Anstelle der Druckdifferenzen können auch Differenzen des aus Druck und Durchflußrate gebildeten Quotienten $Q = \dfrac{p}{F}$ bestimmt und registriert werden.

Eine vorteilhafte Ausführungsform des erfindungsgemäßen Verfahrens ist in Fig. 1 erläutert ; bei dieser Ausführungsform ist Rohr (1) in einem By-pass zur wasserführenden Leitung (6) angeordnet. Mit Hilfe einer vor Rohr (1) in den By-pass eingebauten Pumpe (7) (allgemein : einer Vorrichtung zur Einstellung einer konstanten Durchflußrate F) wird die Stärke des durch Rohr (1) geführten Wasserstromes eingestellt. Ist der auf organische Verbindungen zu überwachende Wasserstrom durch Schwebestoffe, Sand und dergleichen verunreinigt, so wird der in den By-pass eintretende Wasserstrom vorteilhaft durch ein Filter (8) mechanisch gereinigt.

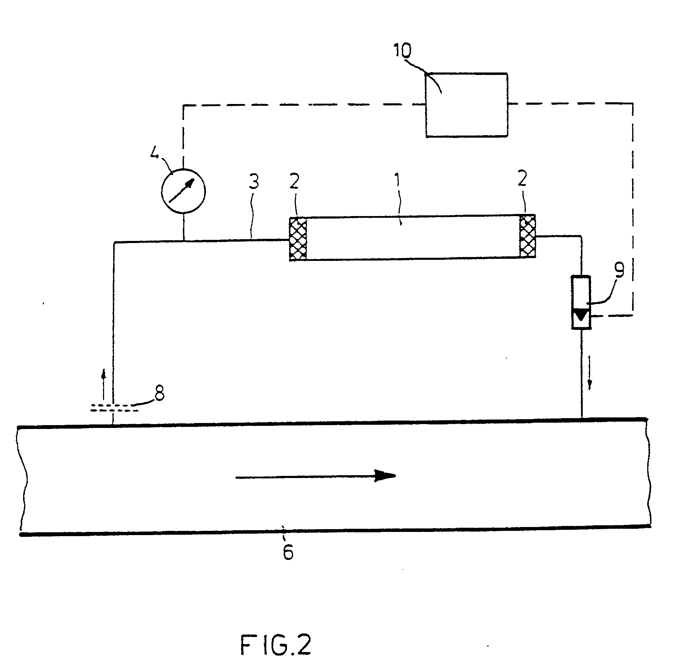

Eine besonders vorteilhafte Ausführungsform des erfindungsgemäßen Verfahrens ist in Fig. 2 erläutert. Bei dieser Ausführungsform wird auf die Pumpe (7) zur Einstellung einer konstanten Durchflußrate verzichtet und statt dessen der Druck der Eintrittsseite und, mit einem Rohr (1) nachgeschalteten Strömungsmesser (9), die Durchflußrate bestimmt.

Bei dieser Ausführungsform wird die durch das Quellen des Signalharzes bewirkte Druckänderung $\Delta$p nicht unmittelbar zur Signalgebung ausgenutzt, sondern die Änderung des aus Eingangsdruck und Durchflußrate gebildeten Quotienten $Q = \dfrac{p}{F}$.

Es wurde gefunden, daß dieser Quotient Q bei konstantem Strömungswiderstand ( = unverändertem Quellungszustand des Signalharzes in Rohr 1) im By-pass unabhängig von etwaigen Schwankungen der Durchflußmenge einen konstanten Wert aufweist. Nur wenn sich der Strömungswiderstand im By-pass (d.h. der Quellungszustand des Signalharzes in Rohr (1)) ändert, ändert sich der Wert des Quotienten Q. Die Änderung von $\Delta$Q läßt sich mit Hilfe eines elektronischen Meßgerätes (10) in einfacher Weise registrieren.

Die Registrierung der Änderung $\Delta$Q hat gegenüber der Registrierung der Änderung des Druckes $\Delta$p den Vorteil, daß sie keinen konstanten Vordruck erfordert. Das Einstellen eines konstanten Vordruckes erfordert eine technisch komplizierte Regelstrecke mit technisch aufwendiger Pumpe. Die Mischung des Eintrittsdrucks und der Durchflußrate ist technisch wesentlich einfacher und daher wirtschaftlicher.

Beispiel 1

Ein 50 mm langes Glasrohr (innerer Durchmesser : 4,5 mm) wird mit 0,79 ml eines Perlpolymerisates aus 98 Gew.-% Styrol und 2 Gew.-% Divinylbenzol (mittlere Korngröße : 0,5 mm) gefüllt. Das Glasrohr ist an beiden Enden mit je einer Glasfritte versehen und wird in der in Fig. 1 erläuterten Meßanordnung angewendet.

Durch dieses Rohr wird ein Wasserstrom, der 200 ppm 2,4-Dichlorbenzylchlorid enthält, mit einer Durchflußrate von 7,5 l/Stunde geleitet. Nach 3 Minuten (entsprechend einem Durchsatz von 0,375 l) steigt der Druck vor dem Rohr von anfänglich 0,7 bar auf 0,8 bar an.

Beispiel 2

Ein 50 mm langes, an beiden Enden mit je einer Glasfritte verschlossenes Glasrohr (innerer Durchmesser: 6 mm) wird mit 1,41 ml des in Beispiel 1 beschriebenen Perlpolymerisates gefüllt. Gearbeitet wird in der in Fig. 1 beschriebenen Meßanordnung.

Durch das Rohr wird ein 2000 ppm 2,4-Dichlorbenzalchlorid enthaltender Wasserstrom mit einer Durchflußrate von 7,5 l/Stunde geleitet. Nach 5 Minuten (entsprechend einem Durchsatz von 0,625 l) ist der Druck von anfänglich 0,3 auf 0,42 bar angestiegen.

Beispiel 3

Gearbeitet wird in der in Fig. 1 beschriebenen Meßanordnung. Je ein Prüfrohr der in Beispiel 2 beschriebenen Größe und mit der in Beispiel 2 beschriebenen Füllung wird von einem Wasserstrom (mit einer Durchflußrate von 7,5 l/h) durchflossen, der 2000 ppm einer der in der folgenden Tabelle angegebenen organischen Verbindungen enthält.

Der Druckanstieg von anfänglich 0,3 bar auf den gewählten Enddruck von 0,4 bar wird innerhalb der in der nachfolgenden Tabelle ebenfalls angegebenen Zeiten erreicht.

## Tabelle

| Im Wasserstrom enthaltene organische Verbindung | Zeit (min) bis zum Erreichen des vorgegebenen Enddruckes von 0,4 bar (= Auslösen eines Alarmzeichens) |
|---|---|
| Benzol | 4 |
| Toluol | 3 |
| p-Xylol | 2 |
| Chlorbenzol | 4 |
| Chlortoluol | 5 |
| o-Dichlorbenzol | 3 |
| 3,4-Dichlorbenzylchlorid | 3 |
| 2,4-Dichlorbenzalchlorid | 3 |
| o-Chlorbenzaldehyd | 4 |
| 2,4-Dichlorbenzoylchlorid | 3 |
| Dichlorethan | 5 |
| Tetrachlorkohlenstoff | 7 |
| Nitrobenzol | 3 |
| Methylisobutylketon | 5 |
| Cyclohexanol | 8 |

### Beispiel 4

Es wird mit der in Fig. 2 beschriebenen Meßanordnung gearbeitet. Ein 50 mm langes, an beiden Enden mit je einer Glasfritte verschlossenes Glasrohr (innerer Durchmesser : 2 mm) wird mit 0,47 ml des in Beispiel 1 beschriebenen Perlpolymerisates gefüllt.

Durch das Rohr wird ein 20 ppm Benzylchlorid enthaltender Wasserstrom mit einer Durchflußrate von 2,5 l/h geleitet. Nach 10 Minuten (entsprechend einem Durchsatz von 0,416 l) ist der Wert des Quotienten Q von anfänglich 0,494 bar/l auf 0,516 bar/l angestiegen. Dieser Anstieg wird elektronisch angezeigt.

Wurde anstelle des 20 ppm Benzylchlorid enthaltenden Wasserstrom ein 1000 ppm Benzylchlorid enthaltender Wasserstrom durch das mit dem Signalharz gefüllte Rohr geleitet, so stieg der Wert des Quotienten Q von anfänglich 0,471 bar/l nach 10 Minuten auf 0,545 bar/l.

## Ansprüche

1. Verwendung von feinteiligen, quellfähigen organischen Polymerisaten als Indikatoren für in Wasserströmen befindliche organische Verbindungen.

2. Verwendung gemäß Anspruch 1, dadurch gekennzeichnet, daß man als quellfähige organische Polymerisate Polymerisate auf Basis von nicht oder nur schwach vernetztem Polyvinylchlorid, Poly(meth)acrylsäureestern oder Polystyrol verwendet.

3. Verwendung gemäß Anspruch 1 oder 2, dadurch gekennzeichnet, daß die feinteiligen Polymerisate eine Teilchengröße von 0,15-1,25 mm aufweisen.

4. Verwendung gemäß Anspruch 1, 2 und/oder 3, dadurch gekennzeichnet, daß die feinteiligen organischen Polymerisate Perlpolymerisate sind.

5. Verwendung gemäß Ansprüchen 1, 2, 3 und/oder 4, dadurch gekennzeichnet, daß als Indikatoren für polare organische Verbindungen mit 0 bis 4 Gew.-% Divinylbenzol, bezogen auf das Gesamtgewicht der Monomeren vernetzte Polystyrol-Perlpolymerisate und für das Erkennen unpolarer organischer Verbindungen mit 0 bis 10 Gew.-% Divinylbenzol, bezogen auf das Gesamtgewicht der Monomeren, vernetzte Polyacrylsäureester langkettiger Alkohole verwendet werden.

6. Verwendung gemäß einem oder mehreren der Ansprüche 1 bis 5, dadurch gekennzeichnet, daß man zur Anzeige die Änderung des Strömungswiderstandes ausnutzt, den ein bestimmtes, vom zu untersuchenden Wasserstrom, bzw. einem Teilstrom desselben, durchflossenes Volumen des Indikatorharzes diesem (Teil)-Strom bietet.

7. Verfahren zum Anzeigen von in Wasserströmen befindlichen organischen Verbindungen, dadurch gekennzeichnet, daß man den zu untersuchenden Wasserstrom oder einen Teilstrom desselben durch ein mit einem quellfähigen organischen Polymerisat gefülltes Rohr leitet, und die durch die Quellung des Polymerisates verursachte Änderung des Druckes in Polymerisat oder die Änderung des Strömungswiderstandes, die das Polymerisat dem Wasserstrom bietet unmittelbar oder mittelbar bestimmt und/oder für eine Signalgebung ausnutzt.

8. Verfahren nach Anspruch 7, dadurch gekennzeichnet, daß der Quotient aus dem Durchmesser D des mit dem quellfähigen organischen Polymerisat gefüllten Rohres und der Stärke des das Rohr durchfließenden Wasserstromes (Durchflußrate F) einen Wert < 1 aufweist.

## Claims

1. Use of fine particles of swellable organic polymers as indicators of organic compounds present in waterflows.

2. Use according to Claim 1, characterised in that polymers based on uncrosslinked or only slightly crosslinked polyvinyl chloride, poly(meth)acrylates or polystyrene are used as the swellable organic polymers.

3. Use according to Claim 1 or 2, characterised in that the fine particles of polymers have a particle size of 0.15-1.25 mm.

4. Use according to Claim 1, 2 and/or 3, characterised in that the fine particles of organic polymers are bead polymers.

5. Use according to Claims 1, 2, 3 and/or 4, characterised in that polystyrene bead polymers crosslinked by 0 to 4% by weight of divinylbenzene, relative to the total weight of the monomers, are used as indicators for polar organic compounds and polyacrylates of long-chain alcohols, crosslinked by 0 to 10% by weight of divinylbenzene, relative to the total weight of the monomers, are used for detecting non-polar organic compounds.

6. Use according to one or more of Claims 1 to 5, characterised in that the change in flow resistance exerted by a defined volume of the indicator resin on the waterflow which is to be investigated, or a part flow thereof, through the resin is utilised for the indication.

7. Method of indicating organic compounds present in waterflows, characterised in that the waterflow which is to be investigated, or a part flow thereof, is passed through a pipe packed with a swellable organic polymer, and the change in pressure in the polymer, caused by the swelling of the polymer, or the change in flow resistance exerted by the polymer on the waterflow is determined directly or indirectly and/or utilised for emitting a signal.

8. Method according to Claim 7, characterised in that the quotient of the diameter D of the pipe packed with the swellable organic polymer and of the intensity of the waterflow passing through the pipe (flow rate F) has a value of < 1,

## Revendications

1. Utilisation de polymères organiques gonflables, en fines particules, en tant qu'indicateurs de la présence de composés organiques dans des courants d'eau.

2. Utilisation selon la revendication 1, caractérisée en ce que l'on utilise en tant que polymères organiques gonflables des polymères à base de chlorure de polyvinyle non réticulés ou peu réticulés, d'esters poly(méth)acryliques ou de polystyrène.

3. Utilisation selon la revendication 1 ou 2, caractérisée en ce que les polymères en fines particules ont une dimension de particule de 0,15 à 1,25 mm.

4. Utilisation selon la revendication 1, 2 et/ou 3, caractérisée en ce que les polymères organiques en fines particules sont des polymères en perles.

5. Utilisation selon les revendications 1, 2, 3 et/ou 4, caractérisée en ce que l'on utilise en tant qu'indicateurs de la présence des composés organiques polaires des polymères en perles du polystyrène réticulés par 0 à 4 % en poids de divinylbenzène par rapport au poids total des monomères, et, pour la détection des composés organiques non polaires, des esters polyacryliques d'alcools à longue chaîne réticulés par 0 à 10 % en poids de divinylbenzène par rapport au poids total des monomères.

6. Utilisation selon une ou plusieurs des revendications 1 à 5, caractérisée en ce que l'on exploite pour la détection la variation de la résistance à l'écoulement offerte par un volume déterminé de la résine indicatrice au passage du courant d'eau soumis à l'examen ou d'une partie de ce courant.

7. Procédé pour détecter la présence de composés organiques dans des courants d'eau, caractérisé en ce que l'on envoie le courant d'eau soumis à l'examen ou une partie de ce courant au travers d'un conduit garni d'un polymère organique gonflable et on détermine directement ou indirectement la variation de pression dans le polymère causée par le gonflement du polymère ou la variation de la résistance à l'écoulement offerte par le polymère au courant d'eau et/ou on les exploite pour produire un signal.

8. Procédé selon la revendication 7, caractérisé en ce que le quotient du diamètre D du conduit garni du polymère organique gonflable et du débit du courant d'eau traversant le conduit (débit F) a une valeur inférieure à 1.

FIG. 1

FIG.2